# EUROPÄISCHE PATENTANMELDUNG

(11) **EP 0 555 607 A1**
(43) Veröffentlichungstag der Anmeldung: **18.08.1993**
(21) Anmeldenummer: 92440146.6
(22) Anmeldetag: 23.12.1992
(51) Int. Cl.: A61G 9/00, A61F 5/455

(54) **Urinal**

(30) Priorität: 03.01.1992 DE 9200049 U; 11.05.1992 CH 1497/92
(71) Anmelder: Maheu, Lucie, F-83700 Saint-Raphael (FR)
(72) Erfinder: Maheu, Lucie, F-83700 Saint-Raphael (FR); Yang, Chong-Rong c/o MEGALUCK CO LTD, Tainan Hsien 722 (TW)
(74) Vertreter: Werner, Guy

(57) **Zusammenfassung**

Ein wegwerfbares Urinal aus Kunststoff besteht aus einem, aus wasserdichter Folie geformten Beutel (1) und aus einem am oberen Rand des Beutels (1) befestigten Anpassungsschnabel (2). Der Anpassungsschnabel (2) ist derart geformt, um die menschliche Genitalzone wasserdicht während dem Urinieren umschliessen zu können. Der Beutel (1) kann wasserdicht und verschliessbar sein oder kann schlauchartig, nach unten offen, geformt sein und dazu dienen, den Urin zu einem Ablauf zu führen.

## Beschreibung

Die vorliegende Erfindung betrifft ein Urinal.

Urinale, in Form von Harngläsern für die Versorgung von bettlägerigen Kranken sind seit langem bekannt. Die Nachteile dieser Glasgefässe liegen insbesondere darin, dass sie platzraubend und zerbrechlich sind und dass ihre Reinigung, die insbesondere in Spitälern sehr sorgfältig vorgenommen werden muss, viel Zeit in Anspruch nimmt.

Ausser für den oben genannten Anwendungsbereich sind diese vorbekannten Urinale kaum zu gebrauchen. Es gibt jedoch andere Notfallssituationen, wo auch gesunde Menschen ein geeignetes Urinal notwendig hätten. Beispielsweise für Flugreisende ergibt sich dann, wenn in kleinen Flugzeugen keine geeigneten Toilettenanlagen vorhanden sind, das Problem, wie und wo sie Urin lassen können. Auch bei hygienedürftigen Toilettenanlagen ergibt sich häufig das Problem, insbesondere für Damen, einem natürlichen Drang zu folgen, ohne dabei mit den verschmutzten Toiletten in Berührung zu kommen.

Die der Erfindung zugrundeliegende Aufgabe besteht deshalb darin, ein Urinal zu schaffen, das in solchen Notlagen zum Urinieren benutzt werden kann, möglichst im Stehen, selbst für Damen, und das ebenfalls, im Heim- oder Spitalgebrauch, für liegende bzw. sitzende Personen die herkömmlichen Urinale ersetzen kann. Die Aufgabe besteht insbesondere darin, ein zum einmaligen Gebrauch bestimmtes, wegwerfbares billiges Urinal zu schaffen, das leicht und diskret, beispielsweise in einer Damenhandtasche, mit sich geführt werden kann.

Erfindungsgemäss löst diese Aufgabe ein Urinal, das aus
einem, aus einem für Flüssigkeit undurchlässigen Material hergestellten Beutel, der ein offenes oberes Ende hat und
einem Anpassungsschnabel, der aus Kunststoff besteht, und an dem offenen oberen Ende des Beutels befestigt ist, wobei der Anpassungsschnabel im wesentlichen die Form eines im Querschnitt langrunden Rohrstückes aufweist, mit einem ersten gekrümmten Wandendabschnitt, einem zweiten gekrümmten Wandendabschnitt, der höher ist als der erste gekrümmte Wandendabschnitt, und mit zwei Seitenwänden, welche den ersten gekrümmten Wandendabschnitt und den zweiten gekrümmten Wandendabschnitt verbinden, wobei die beiden Seitenwände symmetrische, nach oben konkav geformte Ränder aufweisen und wobei der obere Rand des Anpassungsschnabels in Form eines Flansches ausgebildet ist, besteht.

Der Anpassungsschnabel weist demnach nach oben eine Form auf, die das Anpassen des Urinals an den menschlichen Körper während der Benutzung erleichtert. Insbesondere umschliesst der flanschartig ausgebildete obere Rand praktisch wasserdicht die Schamlippen einer Frau während dem Urinieren. Vorzugsweise ist der Anpassungsschnabel aus halbsteifem, etwas biegsamem Kunststoff geformt, um die Anpassung noch zu erleichtern.

Vorzugsweise erstreckt sich der längs des Umfangs des oberen Randes des Anpassungsschnabels ausgebildete Flansch nach aussen, erstreckt sich jedoch oberhalb des ersten gekrümmten Wandendabschnittes halbkreisförmig nach innen. Durch den halbkreisförmigen nach innen gerichteten Flansch wird verhindert, dass Urin aus dem Beutel austreten kann, insbesondere dann, wenn das Urinal von einer Frau benutzt wird. Vorzugsweise wird der Anpassungsschnabel aus halbsteifem, jedoch biegsamem Kunststoff einstückig geformt und wird das lange runde Rohrstück leicht konisch ausgebildet.

Der zum Auffangen des Urins bestimmte Beutel kann ein abgeschlossenes unteres Ende haben. Der in diesem undurchlässigen Beutel enthaltene Urin kann darin problemlos zur Entsorgung weggetragen werden oder gegebenenfalls zu einer Laboranalyse gebracht werden. In dieser Ausführungsform weist der Beutel vorzugsweise unterhalb seines oberen Randes Mittel auf, um ihn wasserdicht abzuschliessen wie zum Beispiel an sich bekannte ineinander einrastbare Streifen, die durch Druck von aussen ineinander einrasten.

In einer anderen bevorzugten Ausführungsform wird in den unten abgeschlossenen Beutel ein Flüssigkeit absorbierendes Material eingelegt. Das in dem Beutel angeordnete Material, beispielsweise ein hochabsorbierendes Polymer, kann einfliessenden Urin im hohen Masse absorbieren. Vorzugsweise wird das hochabsorbierende Material in einen für Flüssigkeit durchlässigen Sack eingefüllt. Ein solcher Sack besteht beispielsweise aus Papierfaser, Zellulosefasern oder Textilfasern, wie sie beispielsweise für Teebeutel benutzt werden. Der durchlässige Sack wird in dem Innenraum des undurchlässigen Beutels eingelegt, so dass er einen grossen Teil dieses Innenraumes einnimmt. Beim Einfliessen des Urins wird er vom absorbierenden Material absorbiert, welches schwillt. Folglich wird der Innenraum des undurchlässigen Beutels durch sozusagen erstarrtem Urin eingenommen, der nicht mehr herausschwappen kann, was den Abtransport des Beutels erleichtert. Das absorbierende Material kann zum Beispiel ein Polymer sein, kann jedoch auch ein schwammartiges Material sein oder ein watteartiges Material sein.

In einer anderen bevorzugten Ausführungsform, die insbesondere für Frauen bestimmt ist, die schmutzige Toiletten nicht in direktem Kontakt benutzen möchten, besitzt der Beutel ein unteres offenes Ende. Der Beutel kann dann zweckmässig in einer sich nach unten verjüngenden Form ausgebildet werden und dient als Urinführungsschlauch zu einem Ablauf, während der Anpassungsschnabel gegen den Leib gepresst wird.

Anhand von Zeichnungen werden zwei Ausführungsbeispiele der Erfindung näher erläutert. Es zeigen :
Figur 1 eine nach unten offene Ausführungsform eines Urinals in einer seitlichen Ansicht in der Perspektive,
Figur 2 dasselbe Urinal in einer Ansicht von hinten und
Figur 3 eine verschliessbare Ausführungsform eines Urinals im Längsschnitt.

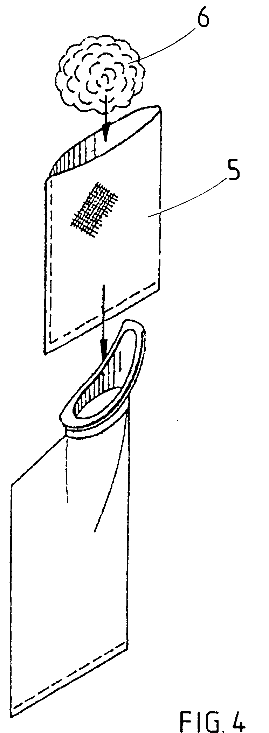

Figur 4 zeigt schematisch in der Perspektive die Anordnung einer unten abgeschlossenen Ausführungsform in die ein durchlässiger Sack mit einem absorbierenden Material eingelegt wird.

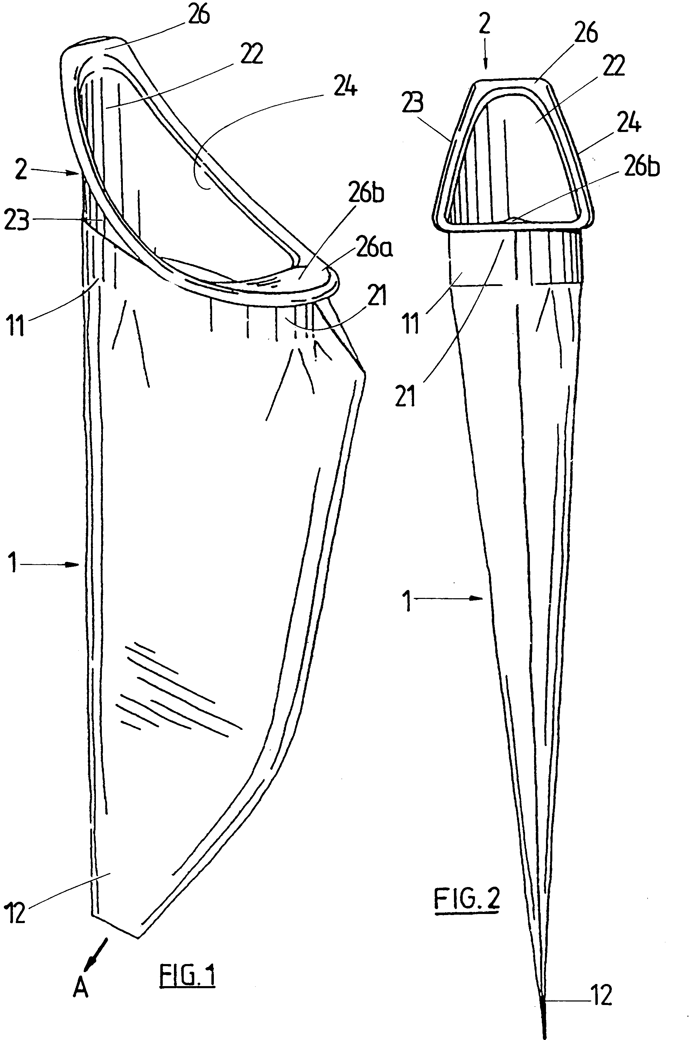

Das in Figur 1 und 2 gezeigte Urinal hat einen aus einer Kunststoffolie, zum Beispiel Polyäthylen, geformten, weichen für Flüssigkeit undurchlässigen Beutel 1. Grob gesehen, weist der Beutel eine Aehnlichkeit mit einem Hemdsärmel auf, der breit an der Schulter und eng am Handgelenk wäre: Der Beutel 1 verjüngt sich von oben nach unten und von hinten nach vorn bis zum unteren offenen Ende 12. Dieses Ende verjüngt sich schlauchartig und ist schräg nach vorne gerichtet. Durch diese besondere Formgestaltung wird das Spritzen nach oben verhindert und das Herausfliessen des Urins nach vorn geführt, in Richtung eines Toiletten- oder sonstigen Ablaufs A.

Die Form des Beutels kann durch entsprechendes Ausschneiden einer Folie, Falten und Versiegeln der Ränder erhalten werden. Eine besondere Form kann auch nur intern vorliegen, indem zum Beispiel einem äusserlich herkömmlich rechteckig geformten, aus einer Mehrschichtfolie mit interner Heissschmelzschicht bestehendem Beutel eine Heisspresse so aufgepresst wird, dass noch ein trichter- oder hornförmiger Kanal für den Durchfluss freibleibt.

An seinem oberen Ende 11 hat der schlauchartige Beutel 1 einen Umfang von zirka 200 bis 250 mm, an seinem unteren offenen Ende 12 hat der Beutel einen Umfang von zirka 20 bis 40 mm. Die Höhe des Beutels beträgt vorzugsweise zirka 200 bis 300 mm, kann aber auch länger sein.

Der Anpassungsschnabel 2 besteht aus Kunststoff und ist mit seinem unteren Abschnitt am offenen oberen Ende 11 des Beutels 1 in bekannter Weise, beispielsweise durch Heisssiegeln befestigt, so dass der Anpassungsschnabel ein Stück mit dem Beutel 1 bildet.

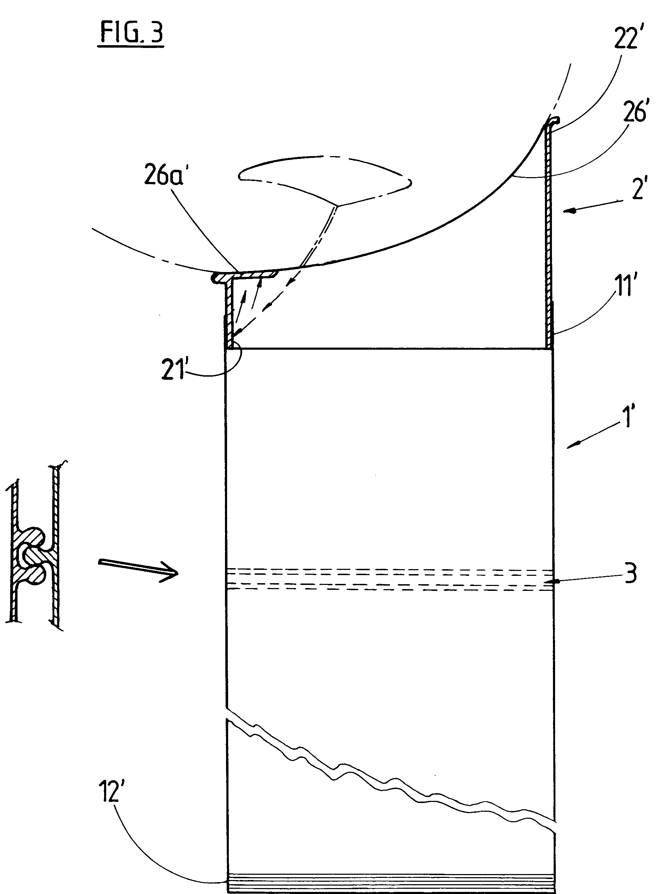

Der Anpassungsschnabel 2 hat einen ersten stark gekrümmten Wandendabschnitt 21, einen zweiten stark gekrümmten Wandendabschnitt 22, der höher liegt als der erste gekrümmte Wandabschnitt 21 und zwei schwach gebogene Seitenwände 23 und 24, welche die beiden gekrümmten Wandendabschnitte 21 und 22 verbinden, wie dies in den Figuren 1 und 2 deutlich gezeigt ist. Längs des Umfangs des oberen Randes des Anpassungsschnabels 2 ist ein, sich nach aussen erstreckender Flansch 26 ausgebildet, dessen Fläche das Anpassen des Anpassungsschnabels 2 an den menschlichen Körper während der Benutzung erleichtert. Der Flansch 26 weist, oberhalb des Wandendabschnittes 21, einen halbkreisförmigen verbreiterten Flanschteil 26a auf. Dieser Flanschteil 26a überdeckt den Wandendabschnitt 21 und erstreckt sich nach innen wie dies in den Figuren 1 und 3 zu erkennen ist. Zur dichteren Anpassung kann der Flanschteil 26a in Längsrichtung eine symmetrische zentrale sattelförmige Erhebung 26b aufweisen.

Der Anpassungsschnabel hat, in Draufsicht, eine Länge (Längsachse) von zirka 100 mm und eine maximale Breite (einschliesslich Flansch) von zirka 50 mm. Der Wandendabschnitt 21 hat eine Höhe von ungefähr 15 mm und der Wandendabschnitt 22 eine Höhe von ungefähr 60 mm. Diese Dimensionen gestatten die Anpassung an die allermeisten menschlichen Anatomien im normalen Zustand.

Figur 3 zeigt eine Ausführungsform mit einem am unteren Ende verschlossenen Beutel 1'. 3 bis 4 cm unterhalb des oberen offenen Randes 11' befinden sich zwei gegenüberliegende zueinanderpassende Verschliessbänder 3 ähnlich denen, die zum Schliessen und Oeffnen von Verpackungsbeuteln benutzt werden.

Die oberen beschriebenen Anpassungsschnäbel können serienmässig aus billigen Kunststoffen wie Polyäthylen oder Polypropylen als Stücke von 10 bis 20 g geformt werden. Um das Daraufstülpen der Beutel zu erleichtern kann das Rohrstück leicht konisch ausgebildet sein. Das Gesamtgewicht eines Urinals nach vorliegender Erfindung beträgt normalerweise weniger als 30g und das Urinal kann zusammengefaltet oder gerollt, eventuell unter einer sterilen Verpackung ohne Aufwand überall transportiert oder gelagert werden.

## Patentansprüche

1. Urinal bestehend aus einem, aus einem für Flüssigkeit undurchlässigen Material hergestellten Beutel (1,1') der ein offenes oberes Ende (11) hat und einem Anpassungsschnabel (2,2') der aus Kunststoff besteht und an dem offenen oberen Ende (11,11') des Beutels (1,1') befestigt ist, wobei der Anpassungsschnabel (2,2') im wesentlichen die Form eines im querschnitt langrunden Rohrstückes aufweist, mit einem ersten gekrümmten Wandendabschnitt (21,21'), einem zweiten gekrümmten Wandendabschnitt (22, 22') der höher ist als der erste gekrümmte Wandendabschnitt (21, 21') und mit zwei Seitenwänden (23, 24, 23', 24'), welche den ersten gekrümmten Wandendabschnitt (21, 21') und den zweiten gekrümmten Wandendabschnitt (22, 22') verbinden, wobei die beiden Seitenwände symmetrische, nach oben konkav geformte Ränder aufweisen und wobei der obere Rand des Anpassungsschnabels in Form eines Flansches (26, 26') ausgebildet ist.

2. Urinal gemäss Anspruch 1, dadurch gekennzeichnet, dass der Flansch (26, 26') sich längs des Umfangs des oberen Randes des Anpassungsschnabels nach aussen erstreckt und sich oberhalb des ersten gekrümmten Wandendabschnittes (21, 21') in Form einer halbkreisförmigen Zone (26a, 26a') nach innen erstreckt und vorzugsweise, dass der halbkreisförmige Flanschteil (26a) eine in Längsrichtung symmetrische, zentrale sattelförmige Erhebung (26b) aufweist.

3. Urinal nach Anspruch 1 oder 2, dadurch gekennzeichnet, dass der Anpassungsschnabel (2) aus halbsteifem, jedoch biegsamen Kunststoff einstückig geformt ist und dass das langrunde Rohrstück leicht konisch ausgebildet ist.

4. Urinal nach einem der Ansprüche 1-3, dadurch gekennzeichnet, dass der Beutel (1) in einer sich nach unten verjüngenden Form ausgebildet ist, dass das untere Ende (12) offen ist, und dass der Beutel (1) am unteren offenen Ende (12) schlauchförmig endet und dass dieses schlauchförmige untere offene Ende schräg nach vorne gerichtet ist.

5. Urinal nach einem der Ansprüche 1-3, dadurch gekennzeichnet, dass der Beutel einen internen, durch Versiegeln erhaltenen Urinkanal aufweist, und dass das untere Ende (12) offen ist.

6. Urinal nach einem der Ansprüche 1-3, dadurch gekennzeichnet, dass der Beutel (1') ein unteres, geschlossenes undurchlässiges Ende (12') hat und unterhalb seines oberen Randes (11') Mittel (3) aufweist, um ihn wasserdicht abzuschliessen.

7. Urinal nach einem der Ansprüche 1-3, dadurch gekennzeichnet, dass der Beutel (1') ein unteres geschlossenes undurchlässiges Ende (12') hat und dass in dem erwähnten Beutel (1') ein Flüssigkeit absorbierendes Material 6 angeordnet ist.

8. Urinal nach Anspruch 7, dadurch gekennzeichnet, dass das Flüssigkeit absorbierende Material in einem durchlässigen Sack 5 eingefüllt ist und vorzugsweise, dass die Dimensionen des durchlässigen Sackes ungefähr dem Innenraum des undurchlässigen Beutels entsprechen.

9. Urinal nach Anspruch 7 oder 8, dadurch gekennzeichnet, dass das absorbierende Material ein schwammartiges oder watteartiges Material ist.

10. Urinal nach einem der Ansprüche 7-9, dadurch gekennzeichnet, dass das absorbierende Material ein Polymer ist.
